# FASCICULE DE BREVET EUROPEEN

(11) **EP 3 389 436 B1**
(45) Date de publication et mention de la délivrance du brevet: **14.08.2019**
(21) Numéro de dépôt: 16808993.6
(22) Date de dépôt: 06.12.2016
(51) Int. Cl.: A45D 34/00

(54) **APPAREIL DE TRAITEMENT DES MATIÈRES KÉRATINIQUES HUMAINES**
DEVICE FOR TREATING HUMAN KERATIN MATERIALS
VORRICHTUNG ZUR BEHANDLUNG VON MENSCHLICHEN KERATINSUBSTANZEN

(30) Priorité: 17.12.2015 FR 1562648
(43) Date de publication de la demande: 24.10.2018
(73) Titulaire: SEB S.A., 69130 Ecully (FR)
(72) Inventeur: MANDICA, Franck, 69340 Francheville (FR); PERNOT, Hélène, 69002 Lyon (FR); SABATTIER, Johan, 69440 Mornant (FR)
(74) Mandataire: Bourrières, Patrice
(86) Numéro de dépôt international: PCT/EP2016/079928
(87) Numéro de publication internationale: WO 2017/102455

(56) Documents cités:
- US-B1- 6 543 950

## Description

La présente invention concerne les appareils de traitement des matières kératiniques humaines, notamment la peau, dans lesquels un produit est appliqué sur celles-ci, à partir d'une recharge présente sur l'appareil.

L'invention concerne plus particulièrement mais non exclusivement les appareils dans lesquels la région traitée est soumise à un courant électrique généré par l'appareil, afin de faciliter l'action du produit distribué par l'appareil et appliqué sur les matières kératiniques.

US 6,543,950 décrit an appareil de traitement des matières kératiniques humaines ayant une poignée et une tête de traitement qui forment un coude.

Il existe un besoin pour à la fois permettre une mise en place et un enlèvement aisé de la recharge et la distribution d'une quantité dosée par l'utilisateur de produit prélevé dans la recharge.

L'invention vise à répondre à cet objectif et elle y parvient grâce à un appareil de traitement des matières kératiniques humaines, comportant une tête de traitement et une poignée pour tenir l'appareil d'une main afin d'appliquer la tête sur les matières kératiniques humaines, notamment la peau, l'appareil présentant un logement pour recevoir une recharge contenant un produit à appliquer, la poignée et la tête de traitement formant un coude, le logement étant orienté sensiblement dans l'axe de la tête de traitement et ouvert dans une direction opposée à la tête de traitement. La recharge est agencée de façon à être actionnée par l'utilisateur pour distribuer une dose de produit.

De préférence, la recharge intègre une pompe actionnée par appui sur la recharge lorsque celle-ci est en place dans son logement.

L'invention permet une mise en place et un enlèvement de la recharge très facile puisque le logement est accessible depuis l'extérieur de l'appareil. Par ailleurs, le fait que la recharge comporte la pompe qui est actionnée par l'utilisateur pour distribuer le produit simplifie la réalisation de l'appareil et réduit le risque qu'un dysfonctionnement de la pompe rende l'appareil inutilisable, puisqu'il suffit dans ce cas de remplacer la recharge.

De préférence, la recharge est montée sur l'appareil de façon à pouvoir se déplacer axialement dans le logement, afin de permettre à l'utilisateur d'actionner la pompe en appuyant sur le fond de la recharge.

L'axe longitudinal du logement peut faire un angle (α) avec l'axe longitudinal de la poignée compris entre 10 et 90°, mieux entre 30 et 90°, ce qui confère de l'ergonomie à l'appareil.

La tête de traitement peut être agencée pour soumettre la peau, dans la région traitée, à un courant électrique, l'appareil étant de préférence un appareil d'électrophorèse.

Le logement peut déboucher dans le coude formé entre la poignée et la tête de traitement.

La recharge peut comporter un embout de distribution du produit contenu à l'intérieur, et l'appareil peut être agencé pour établir une connexion fluidique avec l'embout de distribution lorsque la recharge est en place dans l'appareil, afin de canaliser le produit sortant de la recharge vers un ou plusieurs orifices de distribution sur la tête de traitement.

La recharge fait de préférence saillie hors du logement lorsqu'elle est en place dans celui-ci.

L'invention pourra être mieux comprise à la lecture de la description détaillée qui va suivre, d'un exemple de mise en oeuvre non limitatif de celle-ci, et à l'examen du dessin annexé, sur lequel :
- la figure 1 représente de façon schématique, en perspective, un appareil de traitement selon l'invention, la recharge étant sur le point d'être mise en place dans l'appareil,
- la figure 2 est une vue analogue à la figure 1, la recharge ayant été mise en place dans l'appareil,
- la figure 3 représente isolément la recharge, et
- la figure 4 représente un détail de la recharge de la figure 3.

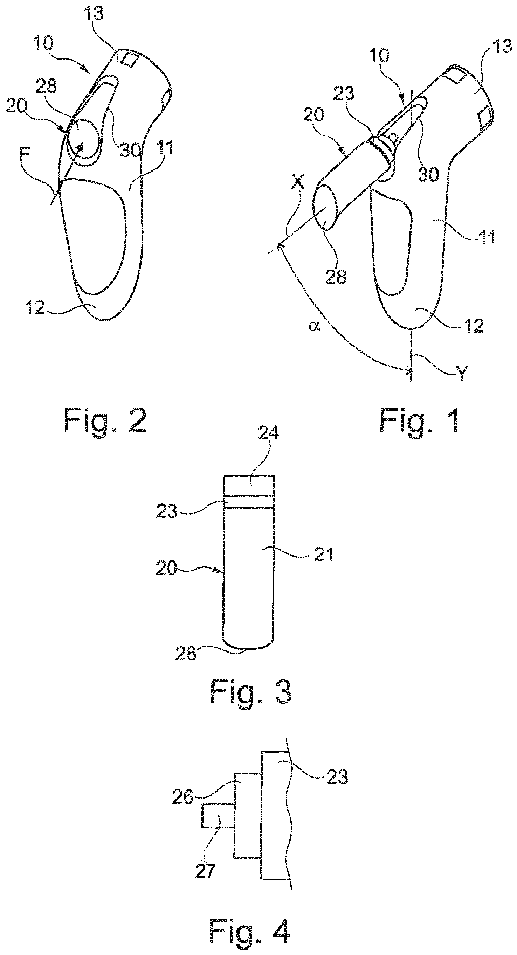

L'appareil de traitement 10 selon l'invention, représenté sur les figures 1 et 2, comporte un boîtier 11 qui présente une forme coudée, avec une poignée 12 destinée à être tenue dans la main de l'utilisateur et une tête de traitement 13 qui s'étend selon un axe X qui fait un angle α avec l'axe longitudinal Y de la poignée. Cet angle α est par exemple compris entre 10 et 90° de façon à permettre une manipulation aisée de l'appareil.

Le boîtier 11 loge un circuit électronique non représenté, qui génère un courant au sein de la région traitée au cours du fonctionnement de l'appareil 10, celui-ci pouvant être un appareil d'électrophorèse. Au moins une électrode, non apparente, peut être placée au contact du produit à l'intérieur de l'appareil, avant sa distribution par la tête de traitement 13. L'appareil peut également comporter une contre-électrode qui est par exemple au contact de la main de l'utilisateur, de façon à permettre l'établissement d'un courant circulant entre l'électrode et la contre-électrode, au sein de la région traitée. De préférence, le produit contenu dans la recharge, qui est de préférence une composition cosmétique ou dermatologique, est conducteur de l'électricité.

Le boîtier 11 peut également loger une source d'énergie électrique telle qu'une ou plusieurs piles ou batteries.

On a représenté isolément à la figure 3 la recharge 20 destinée à être utilisée dans l'appareil et contenant le produit à appliquer.

La recharge 20 comporte un corps 21 formant réservoir et une tête de distribution 23 qui peut être munie avant la première utilisation d'un capuchon de protection 24, lequel est alors enlevé avant la mise en place de la recharge 20 dans l'appareil 10.

On a représenté à la figure 4 un détail de la tête de distribution 23, le capuchon de protection 24 étant enlevé.

La tête de distribution 23 comporte un élément mobile 26 portant une canule de distribution 27 qui s'engage de façon étanche dans un logement correspondant prévu dans la tête de traitement 13, de façon à ce que le produit distribué par la recharge 20 gagne un ou plusieurs orifices de distribution prévus dans la tête de traitement 13.

L'élément 26 actionne un mécanisme de pompe contenu dans la recharge 20, une telle pompe pouvant être similaire à celles équipant les flacons cosmétiques rencontrés sur le marché.

La recharge 20 est reçue dans un logement 30 de l'appareil 10, ce logement étant d'axe longitudinal parallèle à l'axe longitudinal de la tête 13, et ouvert à la fois sur le dessus et vers l'arrière. Le logement 30 débouche ainsi sur la surface extérieure du coude formé par la jonction entre la tête de traitement 13 et la poignée 12.

La recharge 20 est reçue dans le logement 30 avec une possibilité de coulissement selon son axe longitudinal, afin de permettre à l'utilisateur, en appuyant sur le fond 28 de la recharge, de pousser celle-ci vers l'avant et ainsi de provoquer le déplacement de l'élément 26 relativement au corps de la recharge, et donc d'actionner la pompe intégrée à la recharge. Ainsi, l'utilisateur, en exerçant une poussée selon la flèche F de la figure 2, peut distribuer une dose de produit dans la tête de traitement. Lorsque l'utilisateur relâche sa poussée, la recharge est ramenée en arrière par un ressort de rappel intégré à la pompe. L'utilisateur peut ainsi distribuer autant de doses de produit que nécessaire en fonction du traitement effectué, en agissant sur la recharge 20.

L'appareil est avantageusement agencé de façon à détecter un besoin en produit et le signaler par l'émission d'un message visuel, sonore ou vibratoire à l'utilisateur, grâce à un ou plusieurs indicateurs par exemple, non apparents sur les figures. Ainsi, à chaque signal demandant à l'utilisateur de distribuer une dose de produit, celui-ci peut agir sur la recharge 20 comme expliqué précédemment.

Le besoin en produit est par exemple déterminé par une mesure de l'impédance du circuit entre l'électrode et la contre-électrode, laquelle peut renseigner sur la qualité du contact de l'appareil avec les matières kératiniques traitées, une conduction électrique insuffisante pouvant être représentative d'une quantité de produit insuffisante à l'interface entre la tête de traitement et les matières kératiniques traitées.

On peut donner au fond 28 de la recharge 20 une forme ayant un contour non circulaire, notamment elliptique, le fond 28 pouvant s'étendre dans un plan qui est non perpendiculaire à l'axe longitudinal X de la tête de traitement 13, de façon à faciliter l'exercice d'une poussée par l'utilisateur sur le fond 28, notamment avec le pouce. Le fond 28 peut notamment s'étendre sensiblement selon un plan parallèle à l'axe longitudinal Y de la poignée, lorsque la recharge 20 est en place.

L'appareil 10 et la recharge 20 sont avantageusement configurés de telle sorte que dans ce cas la recharge 20 soit disposée de façon orientée dans le logement 30, avec par exemple le grand axe de l'ellipse délimitant le fond 28 de la recharge 20 orienté dans un plan coplanaire à l'axe longitudinal Y de la poignée.

L'invention n'est pas limitée à l'exemple qui vient d'être décrit.

En particulier, la recharge peut comporter une paroi souple sur laquelle l'utilisateur appuie pour distribuer le produit.

## Revendications

1. Appareil de traitement des matières kératiniques humaines, comportant une tête de traitement (13) et une poignée (12) pour tenir l'appareil d'une main afin d'appliquer la tête (13) sur les matières kératiniques humaines, notamment la peau, l'appareil présentant un logement (30) pour recevoir une recharge (20) contenant un produit à appliquer, la poignée et la tête de traitement formant un coude, le logement (30) étant orienté sensiblement dans l'axe de la tête de traitement et ouvert dans une direction opposée à la tête de traitement,
la recharge étant agencée de façon à être actionnée par l'utilisateur pour distribuer une dose de produit.

2. Appareil selon la revendication 1, la recharge intégrant une pompe actionnée en appuyant sur la recharge lorsque celle-ci est en place dans son logement.

3. Appareil selon la revendication 2, la recharge étant montée sur l'appareil de façon à pouvoir se déplacer axialement dans le logement, afin de permettre à l'utilisateur d'actionner la pompe en appuyant sur le fond (28) de la recharge.

4. Appareil selon l'une quelconque des revendications 1 à 3, l'axe longitudinal du logement faisant un angle (α) avec l'axe longitudinal de la poignée compris entre 30 et 90°.

5. Appareil selon l'une quelconque des revendications 1 à 4, la tête de traitement (13) étant agencée pour soumettre la peau, dans la région traitée, à un courant électrique.

6. Appareil selon l'une quelconque des revendications précédentes, le logement (30) débouchant dans le coude formé entre la poignée et la tête de traitement.

7. Appareil selon l'une quelconque des revendications précédentes, la recharge comportant un embout de distribution (7) du produit contenu à l'intérieur et l'appareil étant agencé pour établir une connexion fluidique avec l'embout de distribution lorsque la recharge est en place dans l'appareil, afin de canaliser le produit sortant de la recharge vers un ou plusieurs orifices de distribution sur la tête de traitement.

8. Appareil selon l'une quelconque des revendications précédentes, la recharge (20) faisant saillie hors du logement (30) lorsqu'elle est en place dans celui-ci.

## Patentansprüche

1. Einrichtung zur Behandlung menschlicher Keratinstoffe, die einen Behandlungskopf (13) und einen Griff (12) zum Halten der Einrichtung mit einer Hand enthält, um den Kopf (13) auf die menschlichen Keratinstoffe, insbesondere die Haut, anzulegen, wobei die Einrichtung eine Aufnahme (30) zum Empfangen einer Nachfüllung (20) aufweist, die ein aufzutragendes Produkt beinhaltet, wobei der Griff und der Behandlungskopf ein Winkelstück bilden, wobei die Aufnahme (30) im Wesentlichen in der Achse des Behandlungskopfs ausgerichtet und in eine Richtung entgegengesetzt zu dem Behandlungskopf offen ist,
wobei die Nachfüllung derart eingerichtet ist, dass sie von dem Benutzer zum Verteilen einer Produktdosis betätigt wird.

2. Einrichtung nach Anspruch 1, wobei die Nachfüllung eine Pumpe integriert, die durch Drücken auf die Nachfüllung, wenn diese in ihrer Aufnahme an Ort und Stelle ist, betätigt wird.

3. Einrichtung nach Anspruch 2, wobei die Nachfüllung derart auf die Einrichtung montiert ist, dass sie sich axial in der Aufnahme bewegen kann, um es dem Benutzer zu erlauben, die Pumpe durch Drücken auf den Boden (28) der Nachfüllung zu betätigen.

4. Einrichtung nach einem der Ansprüche 1 bis 3, wobei die Längsachse der Aufnahme einen Winkel (a) mit der Längsachse des Griffs bildet, der zwischen 30 und 90° liegt.

5. Einrichtung nach einem der Ansprüche 1 bis 4, wobei der Behandlungskopf (13) eingerichtet ist, um die Haut in dem behandelten Bereich einem elektrischen Strom zu unterwerfen.

6. Einrichtung nach einem der vorstehenden Ansprüche, wobei die Aufnahme (30) in das Winkelstück, das zwischen dem Griff und dem Behandlungskopf gebildet ist, mündet.

7. Einrichtung nach einem der vorstehenden Ansprüche, wobei die Nachfüllung einen Verteilungsansatz (7) des Produkts, das in dem Inneren eingerichtet ist, enthält, und die Einrichtung eingerichtet ist, um eine fluidische Verbindung mit dem Verteilungsansatz herzustellen, wenn die Nachfüllung in der Einrichtung an Ort und Stelle ist, um das Produkt, das aus der Nachfüllung austritt, zu einer oder mehreren Verteilungsöffnungen auf dem Behandlungskopf zu kanalisieren.

8. Einrichtung nach einem der vorstehenden Ansprüche, wobei die Nachfüllung (20) aus der Aufnahme (30) herausragt, wenn sie in dieser an Ort und Stelle ist.

## Claims

1. Appliance for treating human keratin materials, comprising: a treatment head (13) a handle (12) for holding the appliance in one hand in order to apply the head (13) onto the human keratin materials, notably the skin, and the appliance having a housing (30) for holding a refill (20) containing a product to be applied, the handle and the treatment head forming an elbow, the housing (30) being oriented substantially in line with the treatment head and open in a direction opposite to the treatment head, the refill being arranged so as to be actuated by the user in order to dispense a dose of the product.

2. Appliance according to claim 1, the refill integrating a pump actuated by pressing on the refill when the refill is in place inside its housing.

3. Appliance according to claim 2, the refill being mounted on the appliance such that the refill is movable axially in the housing, in order to permit the user to actuate the pump by pressing on the bottom (28) of the refill.

4. Appliance according to any of the 1 to 3, the longitudinal axis of the housing and a longitudinal axis of the handle forming an angle (α) of between 30 and 90°.

5. Appliance according to any of claims 1 to 4, the treatment head (13) being arranged in order to subject the skin in the treated region to an electric current.

6. Appliance according to any of the preceding claims, the housing (30) leading to the elbow formed between the handle and the treatment head.

7. Appliance according to any of the preceding claims, the refill comprising a dispensing nozzle (7) for dispensing the product contained inside and the appliance being arranged to establish a fluidic connection with the dispensing nozzle when the refill is in place inside the appliance, in order to channel the product coming from the refill to one or more dispensing openings on the treatment head.

8. Appliance according to any of the preceding claims, the refill (20) protruding out of the housing (30) when the refill is placed inside the housing.
